# EUROPEAN PATENT APPLICATION

(11) **EP 2 923 701 A1**
(43) Date of publication of application: **30.09.2015**
(21) Application number: 15159257.3
(22) Date of filing: 16.03.2015
(51) Int. Cl.: A61K 31/4375, A61K 31/4415, A61K 31/51, A61K 36/185, A61P 3/00, A61P 3/06, A61P 3/08, A61P 3/10, A61K 9/28, A23L 1/00, A61K 36/752, A61K 47/14, A61K 47/20, A61K 47/36, A61K 36/67

(54) **Formulation for the treatment and control of metabolic syndrome and correlated disorders**

(30) Priority: 27.03.2014 IT MI20140528
(71) Applicant: Neopharmed Gentili S.r.l., 20143 Milano (MI) (IT)
(72) Inventor: Ferrari, Gianni, 20143 Milan MI (IT); Del Bono, Alessandro, 20143 Milan MI (IT)
(74) Representative: Asensio, Raffaella Consuelo

(57) **Abstract**

Formulation for the treatment and control of metabolic syndrome and disorders thereof, comprising banaba, berberin, vitamin B1 and vitamin B6.

## Description

### FIELD OF THE INVENTION

The present invention concerns a formulation to be used in a supplementing/nutraceutic agent for the treatment and control of metabolic syndrome and disorders thereof comprising banaba, berberin, vitamin B1 and vitamin B6.

### STATE OF THE ART

In the field of medicine, the term metabolic syndrome means a clinical situation of high cardiovascular risk that comprises a series of risk factors that may or may not be associated with symptoms and alterations of some haematochemical parameters that present simultaneously in the individual. These are often related to the lifestyle of the person (overweight, sedentary lifestyle, poor diet) or to pre-existing pathological situations (obesity, hyperglycaemia, hypertension, presence of high cholesterol in the blood - etc.). It affects a high percentage of the world population, mainly the middle-aged and the elderly.

Studies performed confirm that individuals suffering from metabolic syndrome, who do not drastically change their lifestyle, have a high morbidity and mortality rate linked to cardiovascular problems.

In 2005 the International Diabetes Federation reviewed the diagnostic criteria of metabolic syndrome, proposing as a method of identification and classification, the presence in the same patient of at least 2 of the following disorders:
- Fasting glycaemia: over 100 mg/dl stage IFG;
- Hypertension: over 130/85 mm Hg or need for antihypertensive therapy;
- Hypertriglyceridemia: over 150 mg/dl;
- Low HDL cholesterol: 40 mg/dl for males, 50 mg/dl for females or need for lipid modifying therapy,
associated with a waist circumference of over 94 cm for males, 80 cm for females in patients of Caucasian ethnicity (the parameters vary based on the ethnic group).

In addition there is one big risk factor, age, which is decisive from 45 years of age in men and from 55 in women.

High LDL cholesterol levels (LDL-C) represent one of the biggest cardiovascular risk factors and numerous clinical studies have demonstrated that reducing cholesterol levels leads to a significant reduction in cardiovascular risk. Statins represent the class of drugs most used to change LDL-C plasma levels and for the primary and secondary prevention of cardiovascular events (Catapano AL, Reiner Z, De Backer G et al. ESC/EAS Guidelines for the management of dyslipidaemias The Task Force for the management of dyslipidaemias of the European Society of Cardiology (ESC) and the European Atherosclerosis Society (EAS). Atherosclerosis 2011; 217:3-46).

In some patients treatment with statins does not allow the target values of LDL-C to be reached, and therefore they are often prescribed in combination with other lipid modifying drugs to increase therapeutic efficacy (see above). However, a significant percentage of patients treated with statins, estimated to be between 10-15%, presents intolerance to this class of drugs, linked mainly to muscular problems (myopathy), which often makes it necessary for them to be suspended and alternative drugs to be selected that can effectively reduce LDL-cholesterol levels, (Kashani A, Phillips CO, Foody JM et al. Risks associated with statin therapy: a systematic overview of randomized clinical trials. Circulation 2006; 114:2788-97; Tziomalos K, Athyros VG, Karagiannis A, Mikhailidis DP. Management of statin-intolerant high-risk patients. Curr Vase Pharmacol 2010; 8:632-7 and Vandenberg BF, Robinson J. Management of the patient with statin intolerance. Curr Atheroscler Rep 2010; 12:48-57).

The competitive inhibition of the enzyme Hydroxy methyl-glutaryl-CoA reductase (HMG-CoA reductase), which regulates the limiting-stage step of the synthesis of cholesterol at the level of the hepatocytes, leads to a reduction of the synthesis of cholesterol and to a "reflected" increase in LDL receptors at the level of the hepatic cells, stimulated by relative hypocholesterolemia, with consequent further reduction of plasma cholesterol:
it is this main action mechanism through which statins act. Berberin HCl, contained in the plant Berberis Aristata DC is a vegetable alkaloid that possesses various properties, the most important of which is certainly that of lowering cholesterol (Kong W, Wei J, Abidi P et al. Berberin is a novel cholesterol-lowering drug working through a unique mechanism distinct from statins (Nat Med 2004; 10:1344-51) alongside hypoglycaemic activity (Dong H, Wang N, Zhao L, Lu F. Berberin in the treatment of type 2 diabetes mellitus: a systemic review and meta-analysis. Evid Based Complement Alternat Med 2012; 2012:591654); berberin is able to increase the hepatic levels of the LDL receptor both at the level of mRNA (3.5 times) and of protein (2.6 times) (see above Kong et al). The increase in LDLR mRNA levels is mainly due to a post-transcriptional stabilization mechanism of mRNA (See above Kong et al and Abidi P, Zhou Y, Jiang JD, Liu J. Extracellular signal-regulated kinase-dependent stabilization of hepatic low-density lipoprotein receptor mRNA by herbal medicine berberin. Arterioscler Thromb Vasc Biol 2005; 25:2170-6); moreover, berberin increases transcriptional activity of the LDLR promoter (Lee S, Lim HJ, Park JH et al. Berberin-induced LDLR up-regulation involves JNK pathway. Biochem Biophys Res Commun 2007; 362:853-7). Berberin is also able to negatively modulate the expression of PCSK9 (*pro-protein-convertase-subtilisin-kexin-9*) (Cameron J, Ranheim T, Kulseth MA et al. Berberin decreases PCSK9 expression in HepG2 cells. Atherosclerosis 2008; 201:266-7 and Li H, Dong B, Park SW et al. Hepatocyte nuclear factor 1alpha plays a critical role in PCSK9 gene transcription and regulation by the natural hypocholesterolemic compound berberin. J Biol Chem 2009; 284:28885-95), a physiological inhibitor of the LDL receptor that acts by linking the extracellular domain of LDLR and promotes the intracellular degradation thereof, thus resulting in a reduction of the amount of LDLR on the surface of the hepatocytes (Lambert G, Charlton F, Rye KA, Piper DE. Molecular basis of PCSK9 function. Atherosclerosis 2009; 203:1-7). PCSK9 therefore represents a pharmacological target the inhibition of which makes it possible to modulate the circulating levels of LDL-cholesterol. In HepG2 cells, berberin reduces both the levels of mRNA of PCSK9 and the amount of PCSK9 secreted in a dose-and time-dependent manner (See above Li et al and Cameron et al) and simultaneously increases the levels of LDLR mRNA in a dose- and time-dependent manner. On the other hand, statins are able to increase the expression of PCSK9 (Dubuc G, Chamberland A, Wassef H et al. Statins upregulate PCSK9, the gene encoding the proprotein convertase neural apoptosis-regulated convertase-1 implicated in familial hypercholesterolemia. Arterioscler Thromb Vasc Biol 2004; 24:1454-9 and Careskey HE, Davis RA, Alborn WE et al. Atorvastatin increases human serum levels of proprotein convertase subtilisin/kexin type 9. J Lipid Res 2008; 49:394-8), which could reduce the global effect of statins themselves. Indeed, the promoter of the gene PCSK9 contains, exactly like the promoter of the gene LDLR, a functional sequence known as *sterol regulatory element* (SRE) that responds to variations of the intracellular cholesterol levels. The combination of berberin with a statin reduces the statin-mediated induction of the expression of PCSK9 mRNA (see above Cameron et al and Li et al), through a reduction of the expression of the transcription factors involved in the induction of the transcription of the gene *PCSK9,* thus resulting in transcriptional repression (see above Cameron et al). Moreover, when given in combination, berberin and statin induce the expression of LDLR to a greater extent with respect to the single treatments (see above Cameron et al), suggesting that berberin could be an effective supplement to statins.

In hypercholesterolemic subjects oral administration for 3 months of berberin, which is tolerated well and without side effects, reduces the total LDL-C and TG cholesterol plasma levels (see above Kong et al). After this study, which identified berberin as a potential lipid modifying agent, other studies were conducted in populations of patients with different pathologies (diabetes mellitus, hypercholesterolemia, hyperlipidaemia); a recent meta-analysis showed that the administration of berberin produces a significant reduction of the total cholesterol plasma levels (average difference -0.61 mmol/l, range -0.83/-0.39), LDL-C (average difference 0.65 mmol/l TG (average difference -0.50 mmol/l associated with a substantial increase in HDL levels (average difference +0.05 mmol/l) (Dong H, Zhao Y, Zhao L, Lu F. The effects of berberin on blood lipids: a systemic review and meta-analysis of randomized controlled trials. Planta Med 2013; 79:437-46).

This same meta-analysis confirmed the absence of substantial side effects, suggesting that berberin could have beneficial effects in the control of plasma lipid levels (see above Dong et al) Finally, a recent study evaluated the effect of berberin in patients with metabolic syndrome, showing that the administration of berberin induces remission of metabolic syndrome (36%, p=0.037), reduces circumference (p<0.05), TG (p<0.01), systolic pressure (p<0.01) and insulin secretion (p<0.01), and increases insulin sensitivity (p<0.01) Perez-Rubio KG, Gonzalez-Ortiz M, Martinez-Abundis and et al. Effect of Berberin Administration on Metabolic Syndrome, Insulin Sensitivity, and Insulin Secretion. Metab Syndr Relat Disord 2013). Banaba (*Lagerstroemia speciosa L.*) is a plant the leaf extract of which possesses hypoglycaemic activity, as demonstrated in various animal models and *in vitro* (Stohs SJ, Miller H, Kaats GR. A review of the efficacy and safety of banaba (Lagerstroemia speciosa L.) and corosolic acid. Phytother Res 2012; 26:317-24 and Klein G, Kim J, Himmeldirk K et al. Antidiabetes and Anti-obesity Activity of Lagerstroemia speciosa. Evid Based Complement Alternat Med 2007; 4:401-7)

In particular, in genetically diabetic mice, banaba extract has a beneficial effect on the control of glucose plasma levels (Kakuda T, Sakane I, Takihara T et al. Hypoglycemic effect of extracts from Lagerstroemia speciosa L. leaves in genetically diabetic KK-AY mice. Biosci Biotechnol Biochem 1996; 60:204-8) and on obesity (Suzuki Y, Unno T, Ushitani M et al. Antiobesity activity of extracts from Lagerstroemia speciosa L. leaves on female KK-Ay mice. J Nutr Sci Vitaminol (Tokyo) 1999; 45:791-5) This extract showed a hypoglycaemic activity also in subjects with type II diabetes (Judy WV, Hari SP, Stogsdill WW et al. Antidiabetic activity of a standardized extract (Glucosol) from Lagerstroemia speciosa leaves in Type II diabetics. A dose-dependence study. J Ethnopharmacol 2003; 87:115-7). More recently, the administration of an extract containing banaba (as well as ginseng and an extract of white mulberry leaves), which slows down the development of insulin-resistance and of the consequent hyperglycaemia in genetically diabetic mice (Park MY, Lee KS, Sung MK. Effects of dietary mulberry, Korean red ginseng, and banaba on glucose homeostasis in relation to PPAR-alpha, PPAR-gamma, and LPL mRNA expressions. Life Sci 2005; 77:3344-54.), also proved effective in significantly reducing systemic inflammation markers in subjects with low glucose tolerance or type II diabetes (Kim HJ, Yoon KH, Kang MJ et al. A six-month supplementation of mulberry, korean red ginseng, and banaba decreases biomarkers of systemic low-grade inflammation in subjects with impaired glucose tolerance and type 2 diabetes. Evid Based Complement Alternat Med 2012; 2012:735191).

It is known to use an association of banaba and berberin as a supplement for treating metabolic syndrome or a few associated disorders.

Indeed WO2013016741 describes a formulation containing banaba and berberin as dietary supplement in a weight-loss diet, whereas A.F.G. Cicero et al in" Short Term Effect of a Combined Nutraceutical Effect on Insuline Sensitivity and Related Parameters in Subjects with IFG and Metabolic Syndrome. A double Blind, Randomized, Clinical Trial". Abstract of the 5th International Congress on Prediabetes and Metabolic Syndrome it is stated that a nutraceutical containing such an association is suitable for treating subjects suffering from metabolic syndrome. For WO2013016741 the presence of inositol is also essential, since it is said to reduce insulin sensitivity.

Both of the aforementioned formulations in any case foresee the presence of another essential component, i.e. a chromium salt, in particular chromium picolinate, even if at low doses.

Indeed, as can be seen from the extract shown above of Cicero et Al. Chromium picolinate is able to reduce FPG (fasting glycaemia).

Administration for long time periods such as that necessary for such a type of pathology of formulations containing chromium salts can in some particularly sensitive subjects lead to an accumulation of Chromium in the hepatic and renal cells with consequent gastrointestinal and renal disorders.

Chronic use of chromium picolinate could increase the risk of anaemia since the picolinic acid contained in this salt can alter the functionality of the parotid gland (Ann. Pharmacother 1998; 32:428-31).

### SUMMARY OF THE INVENTION

The Applicant has now surprisingly found that it is possible to have nutraceutical supplements/formulations containing an association of banaba and berberin that do not have the aforementioned drawbacks.

The object of the present invention is therefore nutraceutical supplements/formulations containing banaba and berberin in which chromium salt has been replaced with an association of vitamin B1 and Vitamin B6.

Indeed, the clinical trials shown hereafter demonstrate that the formulations object of the present invention proved effective in reducing the total cholesterol and LDL-cholesterol plasma levels after 4 weeks of administration (-9.43% and -14.4%, p<0.05); triglycerides significantly decreased already two weeks after starting to take the supplement (-13.5%, p<0.05); glycaemia was significantly decreased after 4 weeks of treatment (-8%, p<0.05). Cholesterol-HDL plasma levels significantly increased after 4 weeks of treatment with the supplement (+14.3%, p<0.5), an effect that was absent in the placebo group.

### DESCRIPTION OF THE FIGURES

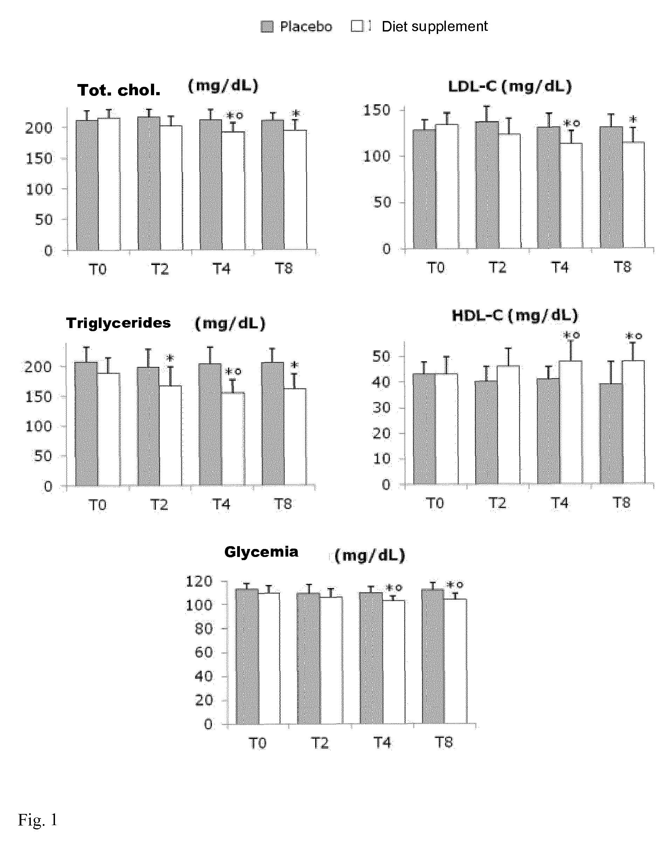

Figure 1 shows the effect of the treatment with placebo or supplement containing berberin and banaba and vitamin B1 and B6 on total cholesterol, LDL and HDL, triglycerides and glycaemia at the start of study (T0) and after 2 (T2), 4 (T4) and 8 (T8) weeks. *p<0.05 vs T0; °p<0.05 vs placebo. The data is expressed as Average± SD.

### DETAILED DESCRIPTION OF THE PRESENT INVENTION

For the purposes of the present invention the term supplements means those specific products aimed at promoting the extraction of certain components or present in a minimal part in the foods of a diet.

For the purposes of the present invention the term nutraceutical means a "food-drug", i.e. a health food that associates nutritional components selected for characteristics such as high digestibility with the curative properties of natural active ingredients of proven and recognised effectiveness.

The supplements/nutraceuticals object of the present invention are formulations in particular in an oral form of the conventional type like for example tablets, capsules, single-doses packets, in which the association of the aforementioned active ingredients is associated with suitable excipients normally used for formulations of such a type, like for example disintegrants, lubricants, dyes, preservatives, etc.) Preferably a single dose of such an oral formulation of weight 0.8 -1.4 g contains from 200 to 800 mg of berberin, preferably in the form of the berberin hydrochloride salt thereof, from 30 to 120 mg of banaba, from 50 to 150% of the recommended daily administration (RDA) of vitamin B1 and from 30 to 100% by weight of the RDA of vitamin B6. Preferably, they are in the form of tablets comprising a core and at least one coating comprising a gastro-resistant film, like for example that obtained with a film based on cellulose derivatives such as ethyl cellulose, isopropyl cellulose, hydroxy propyl methyl cellulose, shellac and other polymers commonly used for this purpose.

According to a particularly preferred solution the tablets with gastro-resistant coating are those prepared as described in international patent application WO2013171270 and identified with the term "enterosomes".

They are characterised in that they contained in the core, as well as the conventional excipients like those indicated above, special excipients capable of promoting the enteral absorption of active ingredients difficult to dissolve in water such as those contained in the supplements/nutraceuticals according to the present invention.

These excipients are polysorbate, N-acetylcysteine (NAC) and chitosan.

Chitosan is a polysaccharide made up of units of N-acetylglucosamine and D-glucosamine, and it can form macrocolloidal gels by protonation of the free amine groups of N-acetylglucosamine followed by the formation of an ammonium polycation.

This polysalification determines the tendency to form gel in water or the relative solubility in water.

NAC acts as an acid capable of protonating chitosan forming a salt with it according to a typical acid-base reaction thus capable of forming only ionic and non-covalent bonds, when the tablet is released in the intestine.

This salt is therefore able to provide chitosan in ionic form, which, since it is watersoluble, acts as mucolytic of intestinal secretions, thus promoting intestinal absorption.

In chitosan salt with N-acetylglucosamine formed in situ, the ponderal ratio between Chitosan and NAC can be between 1:1 and 3:1, in particular between 2:1 and 1: 1 .

The presence of at least one polysorbate in the supplements/nutraceuticals in enterosomic form makes it possible to promote the penetration in circulation of the active ingredients taken orally. Polysorbates are surfactants widely used in the formulation of oil/water emulsion, of tensiolytes and of topical forms. Polysorbates act as absorption promoters exerting a micellization action of the active ingredients that are insoluble or poorly soluble in water and acting as "cellular surfactants", altering the permeability of the cellular membrane. In the formulation of the present invention, the polysorbates thus contribute to making the active ingredient more freely available for intestinal absorption.

The polysorbate comprised in the core of the tablet described of the composition of the present invention can be one of the polysorbates available on the market, like polysorbate 20, 40, 60, 65, 80 and mixtures thereof, and comprised between 20 and 300 mg per single tablet.

Preferably, in the composition according to the invention the polysorbate is polysorbate 80, the composition according to the invention also comprises *Citrus paradisi* extract of content least 40% by weight/total weight of the *Citrus x paradisi* extract in bioflavonoids, and specifically titred in Naringin and Naringenin, and a *Piper nigrum* extract of content 95% by weight/total weight of the *Piper nigrum* extract in piperin. These extracts, in the form of phytocomplex of grapefruit and of black pepper, modulate the activity of different enzymes responsible for the biotransformation of xenobiotics and notably the hepatic and intestinal cytochromes of type P450 and CYP3A.

Indeed, it is known that some substances are able to interact with the P-gp pump located in the external membrane of the enterocyte, inhibiting it. Such an effect reflects in an increased passage in the entero-portal circulation and reduced metabolization carried out by the hepatic and intestinal cytochromes that leads to greater efficacy of the active ingredients taken orally. Such an action mechanism is exerted in particular by some flavonoids contained in the genus Citrus.

Such flavonoidic complexes are also able to inhibit CYP-3A of the enterocyte and in some cases also of the hepatic one, thus reducing the pre-systemic biotransformation. In this way, it is possible to induce an increase in the plasma concentration of the active ingredients contained in the supplement/supplement according to the present invention, which would otherwise be massively biotransformed already before being entered into circulation.

The supplements/nutraceuticals object of the present invention in the form of tablets with gastro resistant coating not in enterosomic form are prepared with dry mixing and possibly wet granulation in water and subsequent dry compression of the mixture or of the possible granulate.

The film-coating of such tablets is generally carried out in a coating pan through spray technology of a solution in organic solvent of the solution containing the gastro-resistant film-coating polymer.

The supplements/nutraceuticals according to the present invention in the form of enterosomes are obtained with the process described in the aforementioned patent document with a process that in particular comprises the following steps:
i.
   i) a mixture of berberin, banaba, vitamin B1 and vitamin B6) is prepared possibly in the presence of excipients of conventional use
   ii) separately, a second mixture is prepared containing chitosan, N-acetylcysteine polysorbate and possibly *Citrus x paradisi* and *Piper nigrum*.
   iii) The mixtures obtained in steps i) and ii) are mixed in a suitable powder mixer;
   iv) A dry granulation is carried out on the mixture obtained through precompression with a suitable machine and subsequent crushing, and sifting
   v) The powder obtained is sent for final compression to obtain a tablet;
   vi) The tablet is then coated through film-coating.
Step vi), i.e. coating the core or tablet obtained in step v) is preferably carried out, with a solution or dispersion in water of an agent capable of forming a gastro-resistant film, or an aqueous dispersion comprising as film-coating polymers ethylcellulose hydroxypropylmethylcellulose and mixtures thereof or an aqueous dispersion of shellac.

According to a particularly preferred solution this coating foresees a first or pre-film coating with an aqueous dispersion of hydroxypropylmethylcellulose and a subsequent film coating with ethyl cellulose.

For illustrating but not limiting purposes the following examples of preparation of the supplements/nutraceuticals according to the present invention are given in the form of conventional gastro-resistant tablets and in the form of enterosomes. Example 1: Preparation of tablets coated with gastro resistant coating weighing 1.1 g.

The following components were mixed for 20 minutes

| | |
|---|---|
| Dry berberis aristata extract | 543,50mg |
| Dry banaba extract | 70,00mg |
| vitamin B1 | 1,70mg |
| vitamin B6 | 1,02mg |
| calcium phosphate | 140,00mg |
| Corn starch | 277,78 mg |
| Polyvinylpyrrolidone | 6,00mg |
| reticulated sodium carboxymethylcellulose | 36,00mg |
| Silica | 12,00mg |
| magnesium stearate | 12,00mg |

The mixture is compressed by means of a suitable compressing machine to obtain a tablet of oval shape weighing 1,100 g characterised by hardness 15-18 kps and disintegration time < 30 minutes.

The film-coating is carried out in a coating pan with an aqueous dispersion containing the following components expressed as % weight over the total weight of the aqueous dispersion.
hydroxypropylmethylcellulose, 44%
yellow iron oxide 4%,
glycerine 2%
water 50%
A yellow-coloured film-coated tablet is obtained.

### EXAMPLE 2 Preparation of enterosome coated tablets

The following mixtures are prepared containing the following components
MIXTURE 1 (unitary composition)

| | |
|---|---|
| Dry berberis aristata extract | 435,00mg |
| Dry banaba extract | 70,00mg |
| vitamin B1 | 1,78mg |
| vitamin B6 | 1,02mg |
| calcium phosphate | 150,00mg |
| Corn starch | 392,30 mg |
| Polyvinylpyrrolidone | 10,00mg |
| reticulated sodium carboxymethylcellulose | 36,00mg |
| Silica | 12,00mg |
| magnesium stearate | 12,00mg |

MIXTURE 2 (unitary composition)

| | |
|---|---|
| Chitosan | 15,00 |
| N-acetyl cystein | 10,00 |
| Grapefruit seeds | 2,00 |
| Black pepper | 2,00 |
| Polysorbate 80 | 1,00 |

Both of the mixtures were prepared after mixing of the respective components for 20 minutes.

### Mixtures 1 and 2 were mixed in a suitable powder mixer.

A dry granulation and a precompression were carried out on the mixture obtained with a suitable machine. The tablets obtained were subjected to crushing, granulation and the powder obtained was sent for final compression in a suitable compressing machine to obtain a tablet of oval shape weighing 1200 mg with hardness 15-18 kps and disintegration time <30 minutes.

Then comes the sub-coating of the tablets in a coating pan using the following aqueous dispersion comprising the following components expressed in % weight over the total weight of the dispersion.

Sepifilm 770 (Hydroxypropylmethylcellulose, cellulose and purified fatty acids)
12%

Water
88%

Thereafter the second gastro-resistant coating is carried out using:
white Surealase clear

(Ethylcellulose, Ammonium hydroxide, medium-chain triglycerides)
34%
white NS Enteric clear
(sodium alginate, purified fatty acids)
1,5% Water:
64,5%

Creamy-yellow-coloured coated tablets were obtained.

### CLINICAL STUDY

In the present study the effect of a food supplement prepared as described in example 1 containing berberin, a banaba extract with thiamine (vitamin B1) and pyridoxine (vitamin B6), administered for 8 weeks on the plasma levels of lipids and of glucose was evaluated in subjects with metabolic syndrome

### Materials and methods

### Study design and participants

A placebo-controlled, double-blind randomized study was carried out, aiming to evaluate the efficacy of a food supplement in the form of a tablet coated with gastro-resistant coating and prepared as shown in example 1.

The subjects of this study were selected from those belonging to the *Centro per lo Statdio dell'Aterosclerosi,* Bassini Hospital, Cinisello Balsamo. The protocol of the study respects the principles of the Helsinki Declaration and was approved by the local Ethics Committee; all of the participants gave their informed consent for the study. 80 subjects were signed up (48 females, 32 males) of age >40 years with metabolic syndrome according to the parameters established by the *Third Report of the National Cholesterol Education Program (NCEP), Expect Panel on Detection, Evaluation and Treatment of High Blood Cholesterol in Adults (Adult Treatment Panel III)* for the definition of metabolic syndrome Grundy SM, Cleeman JI, Daniels SR et al. Diagnosis and management of the metabolic syndrome: an American Heart Association/National Heart, Lung, and Blood Institute Scientific Statement. Circulation 2005; 112:2735-52.

The subjects included had triglycerides (TG) ≥150 mg/dL, HDL ≤40 mg/dL for men and ≤50 mg/dL for women, glycaemia ≥100 mg/dL. The exclusion criteria for this study included the presence of chronic illnesses, the presence of cardiovascular disease and drugs being taken

The subjects signed up were randomised with placebo or with supplement according to a random sequence generated by a computer and were evaluated according to the following times: T-2: two weeks before the start of the study the check the entry conditions; T0: start of the study; T2: two weeks after the start of the study by visit and evaluation of the biochemical and clinical parameters; T4 and T8: four and eight weeks after the start of the study by visit and evaluation of the biochemical and clinical parameters. Of the subjects signed up, 75 completed the treatment (37 in the placebo group and 38 in the supplement group). At the end of the study, the subjects were followed up for another 4 weeks in the absence of supplement/placebo.

### Results

Table 1 shows the baseline characteristics of the subjects participating in the study. There are no differences between the two groups in terms of age, triglyceride plasma level, total cholesterol level, LDL-C and HDL-C and glucose level. During the treatment period no adverse event was reported, and no significant variations of the hepatic (ALT, AST, γ-GT) and muscle (CPK) parameters were observed. Neither of the two groups reported any significant variation of the body mass index (Table 2).

During the study period, in the placebo group no significant variations were observed in the lipid parameters including total cholesterol, LDL-C, HDL, TG, and in glycaemia (Table 2, Figure 1). The administration of the supplement containing berberin and banaba extract, on the other hand, caused a significant effect on the lipid parameters and on glycaemia (Table 2, Figure 1). Indeed, total cholesterol and LDL-C were significantly reduced already after 4 weeks of administration (-9.43% and -14.4%, respectively, with respect to T0, p<0.05) and stayed at these values also after 8 weeks of treatment. The TG plasma levels significantly decreased already two weeks after the start of taking the supplement (-13.5% vs T0, p<0.5), with a further reduction at later times. Glycaemia was also significantly reduced after 4 weeks of treatment (-8% vs T0, p<0.5). The administration of the supplement finally caused a significant increase in HDL plasma levels after 4 weeks of treatment (+14.3% vs T0, p<0.5). Given the low sample size it was not possible to evaluate the effect of the treatment with the supplement containing berberin on HDL in males and females; however, it was possible to see a tendency to increase in both sub-groups (Table 3).

**Table 1. Baseline characteristics of the participants in the study. (Average± SD)**

| **Variable** | **Placebo (n=37)** | **Supplement (n=38)** |
|---|---|---|
| Age | 53±6 | 54±6 |
| Sex (M/F) | 20/17 | 20/18 |
| Total chol., mg/dL | 212±16 | 216±14 |
| LDL-C, mg/dL | 128±11 | 134±13 |
| Triglycerides, mg/dL | 208±25 | 189±26 |
| HDL-C, mg/dL | 43±5 | 43±7 |
| Glycaemia, mg/dL | 113±5 | 110±6 |
| Body mass index, kg/m² | 27.7±1.6 | 27.4±1.7 |

**Table 2. Effect of the supplement containing berberin, banaba extract, Vitamin B1 and Vitamin B6 on the biochemical parameters.(Average± SD)**

| **Variable** | **T-2** | **T0** | **T2** | **T4** | **T8** | **Follow-up (4 weeks)** |
|---|---|---|---|---|---|---|
| **Placebo group** | | | | | | |
| Total chol., mg/dL | 214±13 | 212±16 | 217±13 | 212±17 | 211±12 | 212±14 |
| LDL-C, mg/dL | 134±15 | 128±11 | 137±17 | 131±15 | 131±14 | 131±14 |
| TG, mg/dL | 203±27 | 208±25 | 198±31 | 204±28 | 206±23 | 207±27 |
| HDL-C, mg/dL | 40±7 | 43±5 | 40±6 | 41±5 | 39±9 | 40±8 |
| Glycaemia, mg/dL | 110±7 | 113±5 | 109±8 | 110±5 | 112±6 | 111±8 |
| Body mass index, kg/m² | 27.7±1.6 | 27.6±1.7 | 27.8±1.8 | 27.8±1.7 | 27.7±1.6 | 27.8±1.8 |
| ALT (U/L) | 11+3 | 10+4 | 11+3 | 11+3 | 11+4 | 12+4 |
| AST (U/L) | 15+4 | 14+5 | 16+4 | 14+4 | 15+5 | 15+3 |
| γ-GT (U/L) | 17+5 | 18+6 | 17+4 | 17+5 | 16+5 | 17+6 |
| CPK (U/L) | 70+10 | 75+11 | 68+9 | 71+11 | 72+12 | 74+11 |

| **Supplement group** | | | | | | |
|---|---|---|---|---|---|---|
| Total chol., mg/dL | 212±12 | 216±14 | 203±16 | 192±15*° | 194±17* | 214±13 |
| LDL-C, mg/dL | 132±14 | 134±13 | 123±18 | 113±14*° | 114±16* | 132±15 |
| TG, mg/dL | 193±24 | 189±26 | 167±32* | 155±22*° | 161±25* | 198±27 |
| HDL-C, mg/dL | 42±6 | 43±7 | 46±7 | 48±8*° | 48±7*° | 42±7 |
| Glycaemia, mg/dL | 112±6 | 110±6 | 106±7 | 103±4*° | 104±5*° | 110±7 |
| Body mass index, kg/m² | 27.4±1.7 | 27.3±1.8 | 27.4±1.6 | 27.6±1.8 | 27.3±1.5 | 27.5±1.8 |
| ALT (U/L) | 10+4 | 11+3 | 11+3 | 12+4 | 11+3 | 10+4 |
| AST (U/L) | 16+4 | 15+5 | 15+5 | 14+6 | 14+4 | 15+5 |
| γ-GT (U/L) | 16+6 | 16+5 | 17+6 | 16+5 | 17+5 | 18+6 |
| CPK (U/L) | 74+11 | 71+10 | 72+12 | 69+13 | 71+12 | 73+11 |

| | | | | | | |
|---|---|---|---|---|---|---|
| *p<0.05 vs T0; °p<0.05 vs placebo | | | | | | |

**Table 3.**

| Variation of HDL-C levels (mg/dL) in the 2 male and female sub-groups (Average± | | | | |
|---|---|---|---|---|
| SD) | | | | |
| **Group** | **Male** | | **Female** | |
| | **T0** | **T8** | **T0** | **T8** |
| Placebo (20/17) | 37±7 | 37±6 | 43±6 | 42±7 |
| Supplement (20/18) | 36±8 | 43±9 | 43±7 | 48±9 |

### Discussion

In the present study, the treatment of subjects with metabolic syndrome with a supplement containing berberin and banaba extract with vitamin B1 and Vitamin B6 improved the lipid profile with respect to subjects treated with placebo, having caused a significant reduction in total cholesterol and LDL, in TG and in glycaemia with an accompanying increase in HDL-cholesterol levels. This data suggests that the use of supplements containing natural compounds with substantial activity on some cardiovascular risk factors, such as dyslipidaemia and high levels of glycaemia, can represent a valid alternative in the treatment of subjects without high cardiovascular risk and without clear cardiovascular disease. In particular, the use of berberin in combination with banaba leaf extract and Vitamins B1 and B6 in subjects with metabolic syndrome could be particularly advantageous since it is able to act simultaneously on the levels of glycaemia and on the atherogenic dyslipidaemia that characterises this syndrome, with high levels of TG and LDL-C and low levels of HDL-cholesterol. The advantage would consist of the effective hypoglycaemic and lipid modifying activity without the side effects, at least for short term treatments, ascribable to common hypoglycaemia drugs or statins (Cicero AF, Ferroni A, Ertek S. Tolerability and safety of commonly used dietary supplements and nutraceuticals with lipid-lowering effects. (Expert Opin Drug Safety of commonly used dietary supplements and nutraceuticals with lipid-lowering effects. Expert Opin Drug Saf 2012; 11:753-66).

## Claims

1. A formulation comprising berberin, banaba, vitamin B1 and vitamin B6 in combination with suitable excipients and/or diluents.

2. The formulation according to claim 1 in which berberin is in the form of berberin hydrochloride.

3. The formulation according to claim 1, or 2 for oral use

4. The formulation according to claim 3 comprising from 200 to 800 mg of berberin, from 30 to 120 mg of banaba, from 50 to 150% of the recommended daily administration (RDA) of vitamin B1 and from 30 to 100% by weight of the RDA of vitamin B6.

5. The formulation according to any of the claims 3 or 4 in the form of a tablet comprising a core and an external coating comprising a gastro-resistant film.

6. The formulation according to claim 5, in which the core comprises polysorbate, N-acetylcysteine and chitosan as excipients.

7. The formulation according to claim 6, in which the core comprises *Citrus paradisi* extract and *Piper nigrum* extract.

8. The formulation according to any of the claims 2-6 for supplementing/nutraceutic use for the treatment and the control of the metabolic syndrome and the related disorders.
